# EUROPEAN PATENT SPECIFICATION

(11) **EP 2 938 304 B1**
(45) Date of publication and mention of the grant of the patent: **22.03.2017**
(21) Application number: 13788800.4
(22) Date of filing: 01.10.2013
(51) Int. Cl.: A61F 13/20

(54) **HYGIENIC TAMPON WITH IMPROVED CAPABILITY OF RETAINING A FLUID AND PROCESS OF MANUFACTURING SUCH TAMPON**
HYGIENETAMPON MIT VERBESSERTER FÄHIGKEIT ZUM HALTEN EINER FLÜSSIGKEIT UND VERFAHREN ZUR HERSTELLUNG SOLCH EINES TAMPONS
TAMPON HYGIÉNIQUE À CAPACITÉ DE RÉTENTION DE LIQUIDE AMÉLIORÉE ET SON PROCÉDÉ DE FABRICATION

(30) Priority: 29.12.2012 SI 201200391
(43) Date of publication of application: 04.11.2015
(73) Proprietor: TOSAMA Tovarna sanitetnega materiala d.o.o., 1230 Domzale (SI)
(72) Inventor: ZABRET, Andrej, 1218 Komenda (SI); BRDNIK, Tomaz, 1293 Smarje Sap (SI); CVJETICANIN, Mladen, 1000 Ljubljana (SI)
(74) Representative: Borstar, Dusan
(86) International application number: PCT/SI2013/000057
(87) International publication number: WO 2014/104984

(56) References cited:
- DE-A1-102011 001 282
- US-A1- 2002 120 246
- US-A1- 2005 113 780
- US-A1- 2012 089 111
- US-A1- 2012 283 684

## Description

The invention refers to a tampon, in particular to a hygienic tampon. In accordance with the International Patent Classification, such inventions belong to human necessities, namely to medical science and hygiene, in particular to menstrual tampons and accessories therefor.

By taking into consideration a tampon, which can be manufactured of the same raw materials, by means of the same machines and by means of essentially the same steps like the commercially attractive known tampons, the purpose of the invention is to improve capability of retaining the fluid when inserted into each vaginal cavity and to prevent migration of fluid in both directions, namely from the vaginal cavity and also from outside into said vaginal cavity, wherein simultaneously also the interconnection between the fibers and the withdrawal string should be improved, and disinfecting effect should be achieved.

EP 0 292 831 A1 proposes coating the fibrous band with an adhesive layer in the rear portion of the tampon before winding said fibrous band and obtaining a cylindrical blank for the purposes of compressing thereof into a final shape of a tampon, by which the interconnection between the withdrawal string and fibers should be improved at least by tampons before absorption i.e. before the use. However, said adhesive layer essentially cannot contribute to forming a carrier, and such tampon during absorption intensively expands and maintains its cylindrical shape, so that removing thereof from the vaginal cavity is difficult and due to weak interconnection between the withdrawal string and fibers also pretty risky. In addition to that, the presence of said adhesive layer might have certain impact to mucosa of the vaginal cavity.

A tampon is disclosed in EP 1 458 320 B1, by which the structure of fibers in the front end portion and the rear end portion of the tampon are different, so that expansion of tampon in the area of its rear end portion is much more intensive than on the front end portion, which leads to reduction of leakage of the fluid absorbed in the tampon out from the vaginal cavity. However, in addition to much more complicated manufacturing of such tampon, removal such tampon from the vaginal cavity is difficult and risky, since the interconnection between the withdrawal string and fibrous material of the tampon is weak.

Still further, a tampon is proposed in EP 2 450 013, which is furnished with a means for preventing the tampon to expand in the area of its rear end portion. Said means is either an impregnating coating, in particular of a hydrophobic substance like natural or synthetic wax, vaseline or the like, or a ring or similar accessory placed onto the surface of the tampon. Placing a ring onto the surface of the tampon results in essentially more complicated manufacturing, by which there is also a risk that during absorption and expansion of the tampon, or even later until the tampon is maintained within the vaginal cavity, such ring is unintentionally removed from the tampon, which is then followed by absorption along the complete length of such tampon resulting expansion into usual cylindrical shape. Removal of such moisturized tampon from the vaginal cavity is difficult, and also the interconnection between the withdrawal string and the fibrous material of the tampon is weak, so that said removal is also risky, since upon separating said withdrawal string from the fibrous material, removal of the tampon from the vaginal cavity by means of said string becomes impossible. Also providing said barrier by coating the fibrous material can lead to risk due to essential difference between the state where the fibrous material is impregnated with said hydrophobic substance along its complete cross-section, and the state, where the fibrous material is coated with said hydrophobic substance only on the surface thereof. Simply coating only on the surface namely leads to the effect, which has been discussed in connection with EP 0 292 831 A1. By manufacturing tampons, applying of a hydrophobic substance onto soft fibrous material is pretty complicated and is difficult to be permanently controlled, and in particular, it is difficult to permanently control, whether the fibrous material is really impregnated across its complete cross-section or just coated on its surface. Of this reason, the reliability of performing such process in view of assuring sufficiently high and permanent quality appears to be insufficient.

Further tampons, which are dealing with improved capability of retaining fluid, are disclosed in US 2012/283684 A1, US 2012/089111 A1, US 2002/120246 A1, US 2005/113780 A1 and DE 10 2011 001282 A1.

The previously defined technical problem is solved by means of features, which are included in independent and dependent claims.

The invention will be described in more detail on the basis of embodiment, which is presented in the attached drawings, wherein
- Fig. 1: presents a tampon according to the invention prior to use;
- Fig. 2: presents a tampon according to Fig. 1, in its expanded state due to absorption of each fluid after the use;
- Fig. 3: presents a fibrous belt with compressed barrier during the process of manufacturing of said tampon;
- Fig. 4: is a cross-section along the plane A - A according to Fig. 3; and

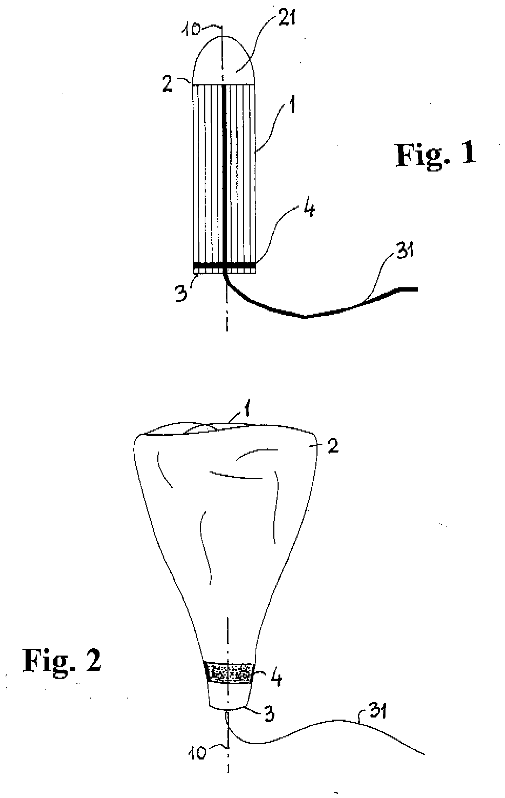

- Fig. 1: schematically presents a step of producing said barrier by simultaneously impregnating thereof.

A tampon with improved capability of retaining a fluid according to the invention is schematically presented in Fig. 1. Such tampon generally consists of a radially compressed cylindrical blank which is formed of appropriately long fibrous band 1 having a pre-determined width and being wound along the longitudinal geometric axis 10 of the tampon, wherein said fibrous band 1 consists of cotton or viscose fibers or of appropriate mixture of cotton and viscose fibers, and wherein the density of said fibrous band 1 is approximately 0,6 g/cm³, by which a desired absorptiveness and capability of expanding during absorption of fluid are assured.

A front end portion 2 of such tampon is adjusted for inserting into a vaginal cavity and is in order to make inserting easier furnished with a gradually narrowed and rounded tip 21, while the rcar end portion 3 of the tampon is furnished with a withdrawal string 3 serving for withdrawing the tampon together with each absorbed fluid from the vaginal cavity.

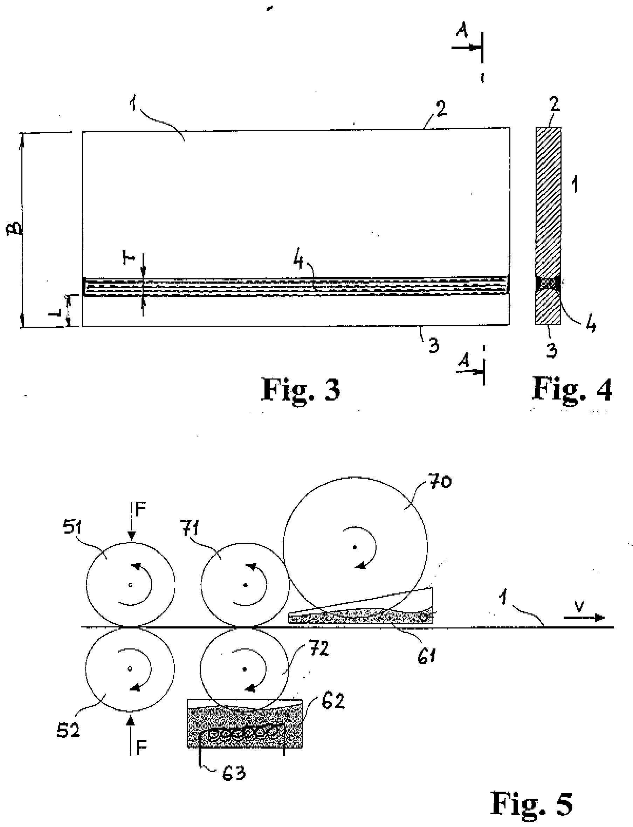

In accordance with the invention, the tampon is on its rear portion 3 furnished with a barrier 4 (Fig. 1), which prevents migration of fluid along the tampon, namely in one or another direction along the geometric axis 10 of the tampon, wherein said barrier 4 is formed by a highly compressed area of said fibrous band 1, which is located at a pre-determined distance L (Fig. 3) apart from the rear end portion 3 of the tampon and in which the density of fibers in said fibrous band 1 is at least approximately three times higher than the density of fibers in residual parts of said fibrous band 1 or tampon, namely at least approximately 2 g/cm³. The width T of said barrier 4 (Fig. 3) is 5 - 30% of the width B of the fibrous band 1 determining the overall length of the tampon. The distance L between said barrier 4 and the rear end portion 2 of the tampon is 0 - 50% of the width B of the fibrous band 1 determining the overall length of the tampon.

As known, absorptiveness of each tampon depends in the density of fibrous band 1, and each tampon is capable to receive certain quantity of fluid per unit of fibrous material. In addition to that, by absorbing each fluid the tampon is also expanding, and removal of such expanded tampon from the vaginal cavity is then much more difficult. Moreover, expansion of tampon in the area of the rear end portion 3 thereof leads to weakening of interconnection between the withdrawal string 31 and fibers in the fibrous band 1, and said withdrawal string 31 can be easily separated from fibers, upon which removal of tampon from the vaginal cavity by means of said withdrawal string 31 becomes impossible.

By forming said barrier 4 with previously mentioned properties and essentially more compressed fibers than in residual areas of the tampon, migration of fluid throughout said barrier 4 is prevented both in a direction out from the vaginal cavity and also in the opposite direction. Since the fluid cannot reach fibers in the area of said barrier 4, expansion in the area of the barrier 4 cannot occur. Consequently, said expansion occurs in the area of the front end portion 2 of the tampon, while expansion on the rear end portion 3 is minimized or does not occur at all. Said hindering of expansion leads to cone-shaped expansion of the tampon when absorbing the fluid during the use, which is less problematic in respect of removal the tampon from the vaginal cavity than a cylindrical shape, by which also a much more reliable interconnection between the withdrawal string 31 and the fibrous band 1 in the area of the rear end portion 3 of the tampon is maintained, so that also removal of the tampon from the vaginal cavity is much more reliable.

In one of possible embodiments, said highly compressed area of fibers in the fibrous band 1 is coated or impregnated with at least one natural or semi-synthetic hydrophobic additive, which is acceptable for vaginal use.

Said natural or semi-synthetic hydrophobic additive is selected from the group which includes bee products, in particular beeswax, honey and a mixture of beeswax and honey. Furthermore, said natural or semi-synthetic hydrophobic additive is selected from the group, which includes essential oils, in particular essential oil obtained from tea-tree (*Melaleuca alternifolia*) leaves in combination with coconut fat, aleuronic acid, essences from grapefruit kernel, extracts of marigold, hydrocotyle, chamomile, sage and garlic. Still further, said natural or semi-synthetic hydrophobic additive is selected from the group, which includes cotton wax, silicone, silicone oils or hard fat, namely semi-synthetic hydrogenated herbal glyceride. Still further, said natural or semi-synthetic hydrophobic additive is selected from the group, which includes fungicides, in particular preparations on the basis of horopito, in particular horopito originating from New Zealand forests. Still further, said natural or semi-synthetic hydrophobic additive is selected from the group, which includes antioxidants, in particular flavonoids taxifolin and quercetin. Still further, said natural or semi-synthetic hydrophobic additive is selected from the group, which includes means for adjusting pH of mucosa, in particular gels on the basis of vitamin C and *aloe vera*. Still further, said natural or semi-synthetic hydrophobic additive is selected from the group, which includes a combination of at least two previously mentioned additives, which is acceptable for vaginal use.

Consequently, application of said additive in the area of said barrier 4 means that simultaneously with forming said barrier 4 also some other effects can be achieved, in particular disinfecting and other preventive effects, which can facilitate menstrual troubles and/or prevent complications.

The invention also provides for a process of manufacturing a tampon with improved capacity of retaining a fluid, wherein
- a fibrous band 1 is formed of woven or non-woven fibers of viscose, or a mixture of cotton and viscose in weight ratio between 50 : 50 to 15 : 85, the pre-determined width B of which defines the overall length of the tampon, wherein the density of fibers in said fibrous band 1 is at least approximately 0,6 g/cm³, by which a desired absorptiveness and expansion by absorbing each fluid is achieved;
- upon which a withdrawal string 31 is placed into the area of the rear end portion 3 and said band 1 is then helically wound around the longitudinal geometric axis 10 of the tampon;
- whereupon such wound cylindrical blank obtained from said fibrous band 1 is exposed to radial compression, by which a front portion 2 with a slightly rounded tip 21 as well as a cylindrical rear portion 3 with integrated withdrawal string 31 are formed.

Process of manufacturing of such tampon is performed in such manner, that immediately after forming said fibrous band 1 and prior to winding thereof around the longitudinal geometric axis 10 of the tampon a barrier 4 having the width T (Fig. 4) is produced at approximately constant distance apart from the rear end portion 3 of the tampon, namely in such manner that said fibrous band 1 is in said area exposed to intensive compression of fibers in such extent that the density of fibers in said area is at least three times greater than the density of fibers in the residual areas of said band 1 and in particular amounts at least approximately 2 g/cm³ which is then followed by winding the band 1 around the longitudinal geometric axis 10 of the tampon and radial compression of the blank consisting of said wound fibrous band 1 for the purposes of providing each desired final shape of the tampon.

Such process is schematically presented in Fig. 5. The fibrous band 1 is with a pre-determined velocity fed between a pair of pressing rollers 51, 52, which compress the fibrous material with a pre-determined force F, by which the fibers are locally, namely in the area of the barrier 4, compressed to the previously mentioned density. This is then optionally followed by winding the band 1 around the longitudinal geometric axis 10, or alternatively, by continuously coating or impregnating by an additive or by a combination of additives.

Alternatively, by producing such barrier 4 consisting of an area with the pre-determined width T and comprising highly compressed fibers, which is located at appropriate distance L apart from the rear end portion 3 of the tampon, fibers of the fibrous band 1 can be optionally coated and/or impregnated in the area of said barrier 4 with at least one natural or semi-synthetic hydrophobic additive, which is selected from the previously mentioned additives, or with a combination of such additives, which is acceptable for vaginal use.

Consequently, applying of two additives is presented in Fig. 5, wherein the first additive is stored in a container 61, and the second additive is stored in a container 62, which is heated by means of a heater 63. Said first additive is applied to the barrier 4 on the bans 1 from the container 61 via a transporting roller 70 and an application roller 71. The second additive is from the heated container 62 and via an application roller 72 applied onto said band 1 from the opposite direction with respect to the previously mentioned first additive.

## Claims

1. Hygienic tampon with improved capability of retaining a fluid, consisting of a radially compressed blank formed of a longitudinal section of a fibrous band (1) having the width (B) and being wound around the longitudinal geometric axis (10) of the tampon, wherein said fibrous band (1) consists of cotton or viscose fibers or a mixture of cotton and viscose fibers, and wherein the density of said fibrous band (1) amounts around 0,6 g/cm³, and wherein such tampon is furnished with a gradually narrowed and slightly rounded tip (21) located at its front end portion (21) for the purposes of facilitating insertion of the tampon into a vaginal cavity, while on the rear end portion (3) a withdrawal thread (31) is foreseen, which is intended for removal the tampon including each fluid absorbed therein from said vaginal cavity, **characterized in that** a barrier (4) is foreseen adjacent to the rear end portion (3) of the tampon, which prevents migration of fluid along the tampon, namely in one or another direction along the longitudinal geometric axis (10) of the tampon, wherein said barrier (4) is formed by means of a highly compressed area on the fibrous band (1), which is located at a distance (L) apart from the rear end portion (3) of the tampon, wherein the density of fibers within said area is at least three times greater than in residual portions of the fibrous band (1) i.e. of the tampon and amounts at least 2 g/cm³.

2. Tampon according to Claim 1, **characterized in that** the width (T) of said barrier (4) corresponds to 5 - 30% of the width (B) of the fibrous band (1) defining the overall length of the tampon.

3. Tampon according to Claim 1, **characterized in that** the distance (L) between said barrier (4) and the rear portion (2) of the tampon corresponds to 0 - 50% of the width (B) of the fibrous band (1) defining the overall length of the tampon.

4. Tampon according to anyone of the preceding Claims, **characterized in that** said highly compressed area of fibers in the fibrous band 1 is coated or impregnated with at least one natural or semi-synthetic hydrophobic additive, which is acceptable for vaginal use.

5. Tampon according to Claim 4, **characterized in that** said natural or semi-synthetic hydrophobic additive is selected from the group which includes bee products, namely beeswax, honey and a mixture of beeswax and honey.

6. Tampon according to Claim 4, **characterized in that** said natural or semi-synthetic hydrophobic additive is selected from the group, which includes essential oils, namely essential oil obtained from tea-tree (*Melaleuca alternifolia*) leaves in combination with coconut fat, aleuronic acid, essences from grapefruit kernel, extracts of marigold, hydrocotyle, chamomile, sage and garlic.

7. Tampon according to Claim 4, **characterized in that** said natural or semi-synthetic hydrophobic additive is selected from the group, which includes cotton wax, silicone, silicone oils or hard fat, namely semi-synthetic hydrogenated herbal glyceride.

8. Tampon according to Claim 4, **characterized in that** said natural or semi-synthetic hydrophobic additive is selected from the group, which includes fungicides, namely preparations on the basis of horopito.

9. Tampon according to Claim 4, **characterized in that** said natural or semi-synthetic hydrophobic additive is selected from the group, which includes antioxidants, namely flavonoids taxifolin and quercetin.

10. Tampon according to Claim 4, **characterized in that** said natural or semi-synthetic hydrophobic additive is selected from the group, which includes means for adjusting *pH* of mucosa, namely gels on the basis of vitamin C and *aloe vera.*

11. Tampon according to anyone of Claims 4 to 10, **characterized in that** said natural or semi-synthetic hydrophobic additive is selected from the group, which includes a combination of at least two additives according to anyone of Claims 5 - 10, which is acceptable for vaginal use.

12. Process of manufacturing a tampon with improved capacity of retaining a fluid, by which a fibrous band (1) is formed of woven or non-woven fibers of viscose, or a mixture of cotton and viscose in weight ratio between 50 : 50 to 15 : 85, the width (B) of which defines the overall length of the tampon, wherein the density of fibers in said fibrous band (1) amounts around 0,6 g/cm³, upon which a withdrawal string (31) is placed into the area of the rear end portion (3) and said fibrous band (1) is then helically wound around the longitudinal geometric axis (10) of the tampon, whereupon such wound cylindrical blank obtained from said fibrous band (1) is exposed to radial compression, by which a front portion (2) with a slightly rounded tip (21) as well as a cylindrical rear portion (3) with integrated withdrawal string (31) are formed, **characterized in that** immediately after forming said fibrous band (1) and prior to winding thereof around the longitudinal geometric axis (10) of the tampon a barrier (4) having the width (T) is produced at approximately constant distance apart from the rear end portion (3) of the tampon, namely in such manner that said fibrous band (1) is in said area exposed to intensive compression of fibers in such extent that the density of fibers in said area is at least three times greater than the density of fibers in the residual areas of said band (1) and amounts at least 2 g/cm³, which is then followed by winding the band (1) around the longitudinal geometric axis (10) of the tampon and radial compression of the blank consisting of said wound fibrous band (1) for the purposes of providing each desired final shape of the tampon.

13. Process according to Claim 12, **characterized in that** by producing said barrier (4) consisting of an area with the pre-determined width (T) and comprising highly compressed fibers, which is located at appropriate distance (L) apart from the rear end portion (3) of the tampon, fibers of the fibrous band (1) in the area of said barrier (4) are coated and/or impregnated with at least one natural or semi-synthetic hydrophobic additive according to anyone of Claims 5-11.

## Patentansprüche

1. Tampon für die Monatshygiene mit verbesserter Fähigkeit zum Zurückhalten eines Fluids, wobei er aus einem radial komprimierten Formling besteht, der aus einem Längsabschnitt eines faserartigen Bandes (1) mit der Breite (B) gebildet ist, welches um die geometrische Längsachse (10) des Tampons gewickelt ist, wobei das faserartige Band (1) aus Baumwolle oder Viskosefasern oder einer Mischung aus Baumwolle und Viskosefasern besteht,
und wobei die Dichte des faserartigen Bandes (1) ungefähr 0,6 g/cm³ beträgt, und wobei ein derartiger Tampon mit einer allmählich sich verjüngenden und leicht abgerundeten Spitze (21) versehen ist, die sich an seinem vorderen Endabschnitt (21) befindet, um das Einführen des Tampons in eine Scheidenhöhle zu erleichtern, wohingegen der hintere Endabschnitt (3) mit einem Rückholfaden (31) versehen ist, welcher dafür bestimmt ist, den Tampon einschließend eines beliebigen Fluids, welches darin aufgenommen wurde, aus der Scheidenhöhle zu entfernen, **dadurch gekennzeichnet, dass** eine Sperre (4) derart vorgesehen ist, dass sie an den hinteren Endabschnitt (3) des Tampon angrenzt, wobei sie die Migration von Fluid entlang des Tampons verhindert, insbesondere in beide Richtungen entlang der geometrischen Längsachse (10) des Tampons, wobei die Sperre (4) mittels eines hochgradig komprimierten Bereichs auf dem faserartigen Band (1) gebildet wird, welcher in einem Abstand (L) von dem hinteren Endabschnitt (3) des Tampons angeordnet ist, wobei die Dichte der Fasern in diesem Bereich mindestens dreimal höher als in den übrigen Abschnitten des faserartigen Bandes (1), d.h. des Tampons, ist und mindestens 2 g/cm³ beträgt.

2. Tampon nach Anspruch 1, **dadurch gekennzeichnet, dass** die Breite (T) der Sperre (4) 5 bis 30 % der Breite (B) des faserartigen Bandes (1) ausmacht, welches die Gesamtlänge des Tampons bestimmt.

3. Tampon nach Anspruch 1, **dadurch gekennzeichnet, dass** der Abstand (L) zwischen der Sperre (4) und dem hinteren Abschnitt (2) des Tampons 0 bis 50 % der Breite (B) des faserartigen Bandes (1) ausmacht, welches die Gesamtlänge des Tampons bestimmt.

4. Tampon nach einem beliebigen der vorhergehenden Ansprüche, **dadurch gekennzeichnet, dass** der hochgradig komprimierte Bereich der Fasern in dem faserartigen Band (1) mit mindestens einem natürlichen oder halbsynthetischen hydrophoben Zusatzstoff beschichtet oder durchtränkt ist, der für eine vaginale Verwendung unbedenklich ist.

5. Tampon nach Anspruch 4, **dadurch gekennzeichnet, dass** der natürliche oder halbsynthetische hydrophobe Zusatzstoff aus der Gruppe ausgewählt ist, die Bienenprodukte umfasst, insbesondere Bienenwachs, Honig und eine Mischung aus Bienenwachs und Honig.

6. Tampon nach Anspruch 4, **dadurch gekennzeichnet, dass** der natürliche oder halbsynthetische hydrophobe Zusatzstoff aus der Gruppe ausgewählt ist, die ätherische Öle, insbesondere das ätherische Öl, welches aus Blättern des Teebaums (*Melaleuca alternifolia*) gewonnen wird, in Kombination mit Kokosfett, Aleuronsäure, Grapefruitkern-Essenzen, Extrakten von Ringelblume, Wassernabel, Kamille, Salbei und Knoblauch umfasst.

7. Tampon nach Anspruch 4, **dadurch gekennzeichnet, dass** der natürliche oder halbsynthetische hydrophobe Zusatzstoff aus der Gruppe ausgewählt ist, die Baumwollwachs, Silikon, Silikonöle oder -Hartfett, insbesondere halbsynthetisches hydriertes Glycerid pflanzlicher Herkunft, umfasst.

8. Tampon nach Anspruch 4, **dadurch gekennzeichnet, dass** der natürliche oder halbsynthetische hydrophobe Zusatzstoff aus der Gruppe ausgewählt ist, die Fungizide, insbesondere Zubereitungen auf der Grundlage von Horopito, umfasst.

9. Tampon nach Anspruch 4, **dadurch gekennzeichnet, dass** der natürliche oder halbsynthetische hydrophobe Zusatzstoff aus der Gruppe ausgewählt ist, die Antioxidantien, insbesondere die Flavonoide Taxifolin und Quercetin, umfasst.

10. Tampon nach Anspruch 4, **dadurch gekennzeichnet, dass** der natürliche oder halbsynthetische hydrophobe Zusatzstoff aus der Gruppe ausgewählt ist, die Mittel zum Einstellen des pH-Werts der Schleimhaut, insbesondere Gele auf der Grundlage von Vitamin C und *Aloe vera*, umfasst.

11. Tampon nach einem beliebigen der Ansprüche 4 bis 10, **dadurch gekennzeichnet, dass** der natürlich oder halbsynthetische Zusatzstoff aus der Gruppe ausgewählt ist, die eine Kombination von mindestens zwei Zusatzstoffen nach einem beliebigen der Ansprüche 5 bis 10 umfasst, welche für eine vaginale Verwendung unbedenklich ist.

12. Verfahren zur Herstellung eines Tampons mit verbesserter Fähigkeit zum Zurückhalten eines Fluids, wobei mittels des Verfahrens (1) ausgehend von webstoff- oder vliesstoffartigen Fasern aus Viskose, oder aus einer Mischung aus Baumwolle und Viskose in einem Gewichtsverhältnis zwischen 50 : 50 und 15 : 85, ein faserartiges Band gebildet wird, dessen Breite (B) die Gesamtlänge des Tampons bestimmt, wobei die Dichte der Fasern in dem faserartigen Band (1) ungefähr 0,6 g/cm³ beträgt, wobei im Bereich von dessen hinterem Endabschnitt (3) ein Rückholfaden (31) angeordnet wird, woraufhin das faserartige Band (1) schraubenartig um die geometrische Längsachse (10) des Tampons gewickelt wird, um den auf diese Weise aus dem faserartigen Band (1) erhaltenen zylindrischen Formling einer radialen Kompression zu unterziehen, mittels welcher ein Vorderabschnitt (2) mit leicht abgerundeter Spitze (21) sowie ein zylindrischer Hinterabschnitt (3) mit integriertem Rückholfaden (31) gebildet werden, **dadurch gekennzeichnet, dass** unmittelbar nach dem Bilden des faserartigen Bandes (1) und bevor dieses um die geometrische Längsachse (10) des Tampons gewickelt wird, eine Sperre (4) mit einer Breite (T) in einem näherungsweise konstanten Abstand vom hinteren Endabschnitt (3) des Tampons geschaffen wird, und zwar insbesondere derart, dass das faserartige Band in diesem Bereich einer so starken Kompression der Fasern ausgesetzt ist, dass die Dichte der Fasern in diesem Bereich mindestens dreimal höher als die Dichte der Fasern in den übrigen Bereichen des Bandes (1) ist und mindestens 2 g/cm³ beträgt, woraufhin das Band (1) um die geometrische Längsachse (10) des Tampons gewickelt wird und der Formling, welcher aus dem gewickelten faserartigen Band (1) besteht, eine radiale Kompression erfährt, um jede beliebige angestrebte Endform des Tampons bereitzustellen.

13. Verfahren nach Anspruch 12, **dadurch gekennzeichnet, dass** durch das Schaffen der Sperre (4), die aus einem Bereich mit vorbestimmter Breite (T) besteht und hochgradig komprimierte Fasern umfasst, wobei sie in einem zweckmäßigen Abstand (L) vom hinteren Endabschnitt (3) des Tampons angeordnet ist, die Fasern des faserartigen Bandes (1) im Bereich der Sperre (4) mit mindestens einem natürlichen oder halbsynthetischen hydrophoben Zusatzstoff nach einem beliebigen der Ansprüche 5 bis 11 beschichtet und/oder durchtränkt werden.

## Revendications

1. Tampon hygiénique possédant une capacité améliorée de rétention d'un fluide, constitué par une ébauche comprimée en direction radiale qui se compose d'un tronçon longitudinal d'une bande fibreuse (1) possédant la largeur (B) et enroulé autour de l'axe géométrique longitudinal (10) du tampon, dans lequel ladite bande fibreuse (1) est constituée par des fibres de coton ou de viscose ou par un mélange de fibres de coton et de viscose, et dans lequel la masse volumique de ladite bande fibreuse (1) s'élève à environ 0,6 g/cm³, et dans lequel le tampon en question est muni d'une extrémité (21) légèrement arrondie et qui se rétrécit progressivement, disposée à sa portion terminale avant (21), ayant pour objet de faciliter l'insertion du tampon dans une cavité vaginale, tandis que l'on prévoit sur la portion terminale arrière (3) un fil de retrait (31) qui est destiné au retrait du tampon, dans lequel est englobé chaque fluide à l'état absorbé, à partir de ladite cavité vaginale, **caractérisé en ce qu'**on prévoit une barrière (4) en position adjacente à la portion terminale arrière (3) du tampon qui empêche une migration du fluide le long du tampon, plus précisément dans l'une ou l'autre direction le long de l'axe géométrique longitudinal (10) du tampon, ladite barrière (4) étant obtenue au moyen d'une zone fortement comprimée sur la bande fibreuse (1), qui est disposée à une certaine distance (L) par rapport à la portion terminale arrière (3) du tampon, la masse volumique des fibres au sein de ladite zone étant au moins trois fois supérieure à celle des portions résiduelles de la bande fibreuse (1), c'est-à-dire du tampon, et s'élevant à au moins 2 g/cm³.

2. Tampon selon la revendication 1, **caractérisé en ce que** la largeur (T) de ladite barrière (4) correspond à une valeur de 5 à 30 % de la largeur (B) de la bande fibreuse (1) définissant la longueur globale du tampon.

3. Tampon selon la revendication 1, **caractérisé en ce que** la distance (L) entre ladite barrière (4) et la portion arrière (2) du tampon correspond à une valeur de 0 à 50 % de la largeur (B) de la bande fibreuse (1) définissant la longueur globale du tampon.

4. Tampon selon l'une quelconque des revendications précédentes, **caractérisé en ce que** ladite zone de fibres fortement comprimée dans la bande fibreuse (1) est enduite ou imprégnée avec au moins un additif hydrophobe naturel ou semi-synthétique qui est acceptable pour son utilisation vaginale.

5. Tampon selon la revendication 4, **caractérisé en ce que** ledit additif hydrophobe naturel ou semi-synthétique est choisi parmi le groupe qui englobe des produits d'abeilles, plus précisément de la cire d'abeille, du miel et un mélange de cire d'abeille et de miel.

6. Tampon selon la revendication 4, **caractérisé en ce que** ledit additif hydrophobe naturel ou semi-synthétique est choisi parmi le groupe qui englobe des huiles essentielles, plus précisément de l'huile essentielle que l'on obtient à partir des feuilles de l'arbre à thé (*Melaleuca alternifolia*) en combinaison avec de la graisse de coco, de l'acide hyaleuronique, des essences de pépins de pamplemousse, des extraits de soucis, un hydrocotyle, de la camomille, de la sauge et de l'ail.

7. Tampon selon la revendication 4, **caractérisé en ce que** ledit additif hydrophobe naturel ou semi-synthétique est choisi parmi le groupe qui englobe de la cire de coton, du silicone, des huiles de silicone ou de la graisse solide, plus précisément du glycéride semi-synthétique hydrogéné à base de plantes.

8. Tampon selon la revendication 4, **caractérisé en ce que** ledit additif hydrophobe naturel ou semi-synthétique est choisi parmi le groupe qui englobe des fongicides, plus précisément des préparations à base d'horopito.

9. Tampon selon la revendication 4, **caractérisé en ce que** ledit additif hydrophobe naturel ou semi-synthétique est choisi parmi le groupe qui englobe des antioxydants plus précisément les flavonoïdes taxifoline et quercétine.

10. Tampon selon la revendication 4, **caractérisé en ce que** ledit additif hydrophobe naturel ou semi-synthétique est choisi parmi le groupe qui englobe des moyens pour régler le pH de la muqueuse, plus précisément des gels à base de vitamines C et d'aloe vera.

11. Tampon selon l'une quelconque des revendications 4 à 10, **caractérisé en ce que** ledit additif hydrophobe naturel ou semi-synthétique est choisi parmi le groupe qui englobe une combinaison d'au moins deux additifs selon l'une quelconque des revendications 5 à 10, qui sont acceptables pour leur utilisation vaginale.

12. Procédé de fabrication d'un tampon possédant une capacité améliorée de rétention d'un fluide, par lequel on forme une bande fibreuse (1) à partir de fibres de viscose tissées ou non-tissées ou d'un mélange de coton et de viscose dans un rapport pondéral entre 50 : 50 et 15 : 85, dont la largeur (B) définit la longueur globale du tampon, dans lequel la masse volumique des fibres dans ladite bande fibreuse (1) s'élève à environ 0,6 g/cm³ ; après quoi, on place un cordon de retrait (31) dans la zone de la portion terminale arrière (3) et on soumet ensuite ladite bande fibreuse (1) à un enroulement hélicoïdal autour de l'axe géométrique longitudinal (10) du tampon ; on expose ensuite une telle ébauche cylindrique enroulée que l'on obtient à partir de ladite bande fibreuse (1) à une compression radiale, si bien que l'on obtient une portion avant (2) possédant une extrémité légèrement arrondie (21), ainsi qu'une portion arrière (3) de forme cylindrique dans laquelle est intégré un cordon de retrait (31), **caractérisé en ce que**, directement après la formation de ladite bande fibreuse (1) et avant son enroulement autour de l'axe géométrique longitudinal (10) du tampon, on procure une barrière (4) possédant une largeur (T) à une distance approximativement constante par rapport à la portion terminale arrière (3) du tampon, plus précisément d'une manière telle que l'on expose ladite bande fibreuse (1) dans ladite zone à une compression intense des fibres de telle sorte que la masse volumique des fibres dans ladite zone est au moins trois fois supérieure à la masse volumique des fibres dans les zones résiduelles de ladite bande (1) et s'élève à au moins 2 g/cm³, ladite exposition étant suivie d'un enroulement de la bande (1) autour de l'axe géométrique longitudinal (10) du tampon et d'une compression radiale de l'ébauche constituée de ladite bande fibreuse enroulée (1) dans le but d'obtenir chaque configuration finale désirée du tampon.

13. Procédé selon la revendication 12, **caractérisé en ce que**, au cours de la production de ladite barrière (4) constituée d'une zone possédant la largeur prédéterminée (T) et comprenant des fibres fortement comprimées, qui est disposée à une distance appropriée (L) par rapport à la portion terminale arrière (3) du tampon, des fibres de la bande fibreuse (1) dans la zone de ladite barrière (4) sont enduites et/ou imprégnées avec au moins un additif hydrophobe naturel ou semi-synthétique selon l'une quelconque des revendications 5 à 11.
